# EUROPEAN PATENT SPECIFICATION

(11) **EP 3 178 456 B1**
(45) Date of publication and mention of the grant of the patent: **15.08.2018**
(21) Application number: 16176782.7
(22) Date of filing: 29.06.2016
(51) Int. Cl.: A61F 13/472, A61F 13/535, A61F 13/539, A61F 13/47, A61F 13/53

(54) **SANITARY NAPKIN**
DAMENBINDE
SERVIETTE HYGIÉNIQUE

(30) Priority: 07.12.2015 TW 104140895
(43) Date of publication of application: 14.06.2017
(73) Proprietor: Chien, Yuan-Cheng, Kaohsiung City 83158 (TW)
(72) Inventor: Chien, Yuan-Cheng, Kaohsiung City 83158 (TW)
(74) Representative: Becker Kurig Straus

(56) References cited:
- WO-A1-02/28335
- GB-A- 2 306 333
- US-A1- 2003 153 232
- US-A1- 2014 358 106

## Description

The disclosure relates to a feminine hygiene product, more particularly to a sanitary napkin.

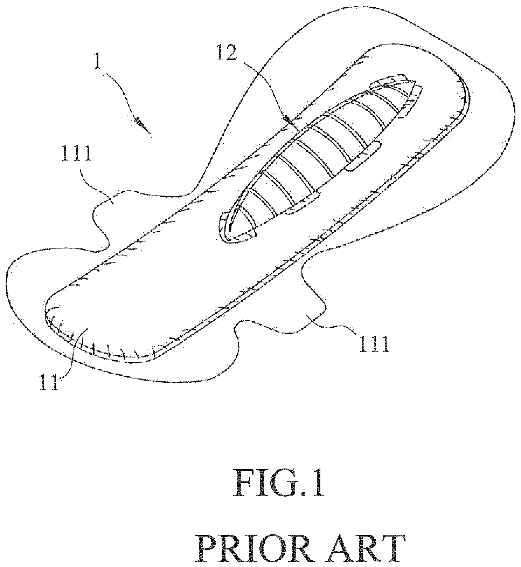

Referring to Fig. 1, a conventional sanitary napkin 1 is shown to include a substrate 11 formed with two side flaps 111, and an absorbent body 12 mounted protrudingly on the substrate 11.

Use of the absorbent body 12 mounted protrudingly on the substrate 11 allows the sanitary napkin 1 to fittingly contact a user's skin so as to absorb secretions quickly and to prevent leakage of secretions from the sanitary napkin. Since the absorbent body 12 is in close contact with the substrate 11, and when used, the absorbent body 12 also fits closely against the user's skin, the sanitary napkin 1 thus has poor air permeability. Use of the conventional sanitary napkin for a long period of time may cause strange odors to be emitted, and comfort of use to be adversely affected.

US 2014/358106 A1 discloses a hygiene pad comprising a series of embossed channels visible when viewing the pad from a user-facing surface.

US 2003/153232 A1 discloses an absorbent article comprising cellulose fibers present at least partially in the form of granules.

Therefore, an object of the disclosure is to provide a sanitary napkin that can alleviate at least one of the drawbacks of the prior art.

The invention provides a sanitary napkin according to claim 1.

According to the disclosure, the sanitary napkin includes: a substrate having a base layer and an absorbent body mounted protrudingly on the base layer, and defining a longitudinal direction and a width direction perpendicular to the longitudinal direction; and at least one absorbing unit including a plurality of connecting portions connected to the absorbent body of the substrate and spaced apart among one another in the longitudinal direction, an absorbing portion extending along the longitudinal direction and connected to the connecting portions, and a plurality of cotton fluffs disposed on the absorbing portion. The absorbent body of the substrate, the connecting portions and the absorbing portion cooperatively define a plurality of aerated channels each extending along the width direction.

Other features and advantages of the disclosure will become apparent in the following detailed description of the embodiments with reference to the accompanying drawings, of which:
Fig. 1 is a perspective view illustrating a conventional sanitary napkin;
Fig. 2 is a perspective view illustrating a first embodiment of a sanitary napkin according to the disclosure;
Fig. 3 is a side view of the first embodiment;
Fig. 4 is a sectional side view of the first embodiment;
Fig. 5 is a perspective view illustrating a variation of the first embodiment;
Fig. 6 is a side view illustrating the variation of the first embodiment;
Fig. 7 is a sectional side view illustrating the variation of the first embodiment;
Fig. 8 is a perspective view illustrating a second embodiment of a sanitary napkin according to the present disclosure;
Fig. 9 is a top view illustrating a third embodiment of a sanitary napkin according to the disclosure;
Fig. 10 is a side view of the third embodiment; Fig. 11 is a perspective view illustrating a fourth embodiment of a sanitary napkin according to the disclosure; and
Fig. 12 is a front view of the fourth embodiment.

Before the disclosure is described in greater detail, it should be noted that where considered appropriate, reference numerals or terminal portions of reference numerals have been repeated among the figures to indicate corresponding or analogous elements, which may optionally have similar characteristics.

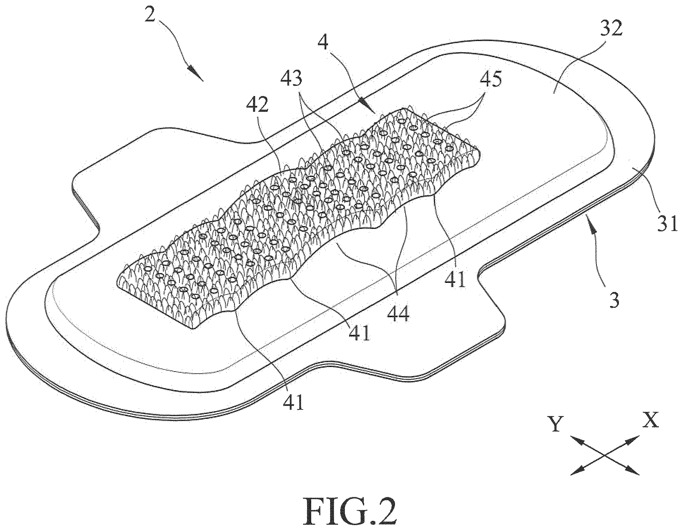

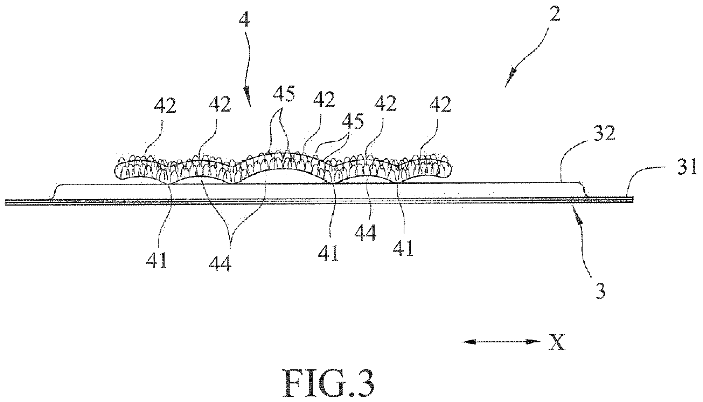

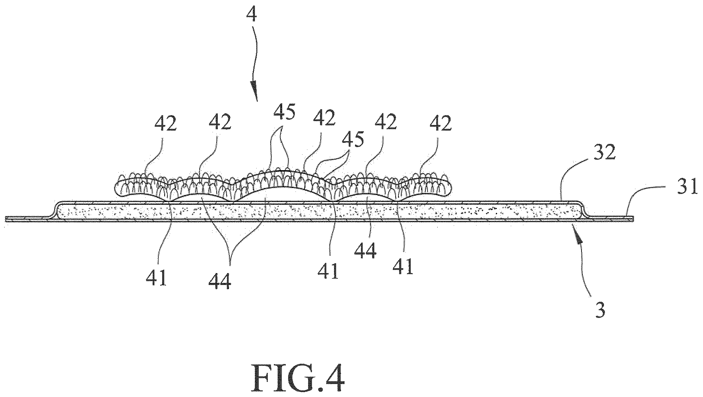

Referring to Figs. 2 to 4, a first embodiment of a sanitary napkin 2 according to the disclosure is shown to include a substrate 3 and an absorbing unit 4 which is in the form of a sheet.

The substrate 3 has a base layer 31 and an absorbent body 32 mounted protrudingly on the base layer 31, and defines a longitudinal direction (X) and a width direction (Y) perpendicular to the longitudinal direction (X).

The absorbing unit 4 includes a plurality of connecting portions 41 connected to the absorbent body 32 of the substrate 3 and spaced apart among one another in the longitudinal direction (X), an absorbing portion 42 extending along the longitudinal direction (X) and connected to the connecting portions 41, and a plurality of cotton fluffs 45 disposed on the absorbing portion 42. In other words, the absorbing portion 42 is connected to the absorbent body 32 of the substrate 3 through the connecting portions 41. The absorbing portion 42 may be formed as parts that are each arcuate in shape. The absorbent body 32 of the substrate 3, the connecting portions 41 and the absorbing portion 42 cooperatively define a plurality of aerated channels 44 each extending along the width direction (Y).

The absorbing unit 4 is formed with a plurality of perforations 43, each spaced apart among one another, extending through the absorbing portion 42, and disposed in fluid communication with the aerated channels 44. In other words, a plurality of the perforations 43 extend through the absorbing portion 42 and are disposed in fluid communication with the aerated channels 44 that are formed under the absorbing portion 42.

It should be noted that two opposite ends of the absorbing unit 4 in the longitudinal direction (X) may be connected to or spaced apart from the absorbent body 32. In this embodiment, the two opposite ends of the absorbing unit 4 are spaced apart from the absorbent body 32.

In this embodiment, the absorbing portion 42 and the plurality of cotton fluffs 45 disposed on the absorbing portion 42 provide the effects of absorption and diffusion of the secretions, and the perforations 43 and the aerated channels 44 improve air-permeability of the sanitary napkin 2 so as to prevent the generation of strange odors and to promote comfort of use.

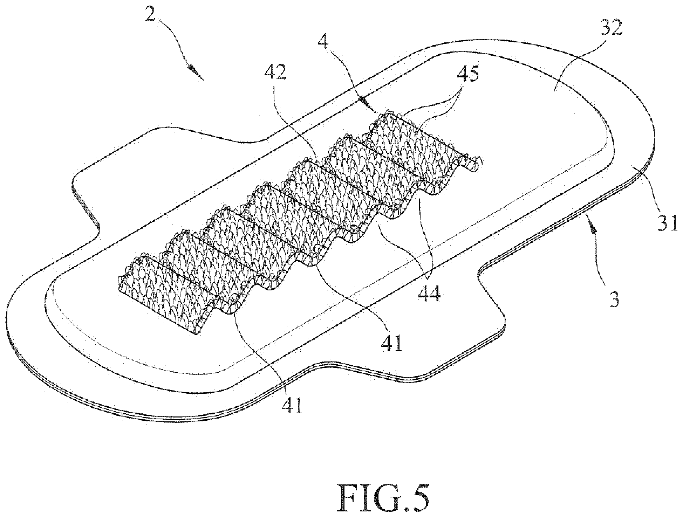

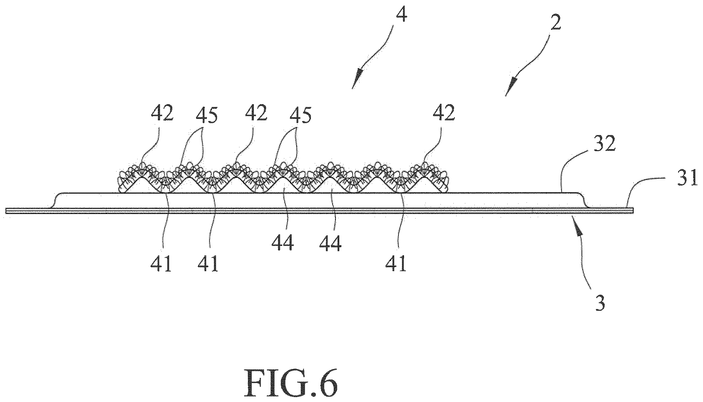

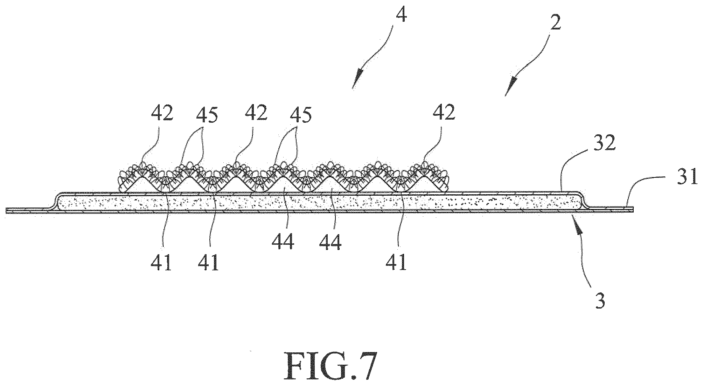

Referring to Figs. 5 to 7, a variation of the first embodiment is shown to be nearly identical to the first embodiment, with the difference being that the absorbing portion 42 extends along the longitudinal direction in the shape of a wave, with peaks protruding away from the absorbent body 32 of the substrate 3 and valleys indented toward the absorbent body 32 of the substrate 3. In this variation of the first embodiment, Figs. 6 and 7 illustrate that the two opposite ends of the absorbing unit 4 are connected to the absorbent body 32.

The absorbing unit 4 may further include a plurality of rows of cotton fluff balls (not shown) disposed on the absorbing portion 42, and the diameters of the cotton fluff balls in one of the rows are different from the diameters of the cotton fluff balls in an adjacent one of the rows.

The cotton fluff balls promote the effect of absorption and diffusion of the secretions. Moreover, since the diameters of the cotton fluff balls in any two adjacent rows are different, there are many gaps among the cotton fluff balls 46, which improve air permeability so as to prevent the generation of strange odors and thereby promote comfort of use.

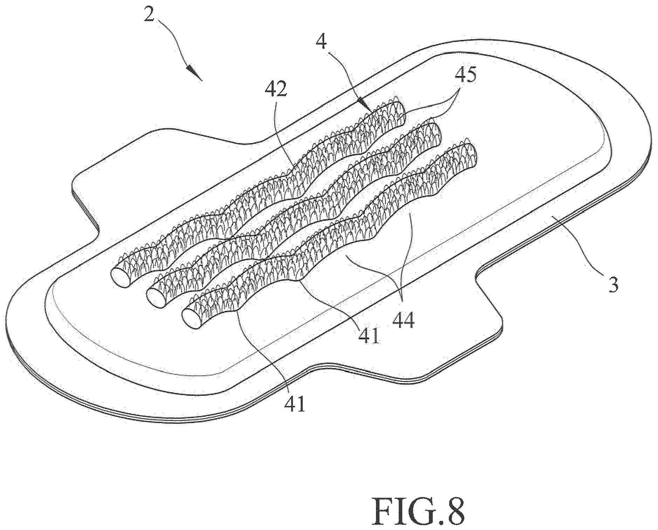

Referring to Fig. 8, a second embodiment of a sanitary napkin 2 according to this disclosure includes a substrate 3 and a plurality of absorbing units 4 that are spaced among one another along the width direction (Y). Each of the absorbing units 4 has an elongated strip shape and extends along the longitudinal direction (X).

In the second embodiment, each of the absorbing units 4 includes a plurality of connecting portions 41 connected to the absorbent body 32 of the substrate 3 and spaced apart among one another in the longitudinal direction (X), an absorbing portion 42 extending along the longitudinal direction (X) and connected to the connecting portions 41, and a plurality of cotton fluffs 45 disposed on the absorbing portion 42. The absorbing portion 42 of each of the absorbing units 4 may be formed as parts that are each arcuate in shape. The connecting portions 41 and the absorbing portion 42 of each of the absorbing units 4 cooperate with the absorbent body 32 of the substrate 3 to define a plurality of aerated channels 44 each extending along the width direction.

With the absorbing units 4 being spaced apart along the width direction (Y), and the aerated channels 44, the sanitary napkin 2 has improved air permeability so as to prevent the generation of strange odors and promote comfort of use. Moreover, with the plurality of cotton fluffs 45 disposed on the absorbing portion 42, the sanitary napkin 2 may fit closely against a user's genitals so as to improve absorption and diffusion properties.

It should be noted herein that, two ends of each of the absorbing units 4 may be connected to the absorbent body 32 of the substrate 3 or may be configured to be spaced apart from the substrate 3.

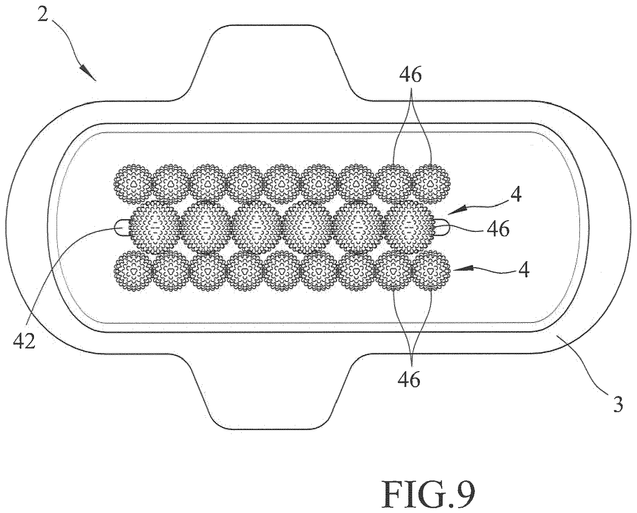

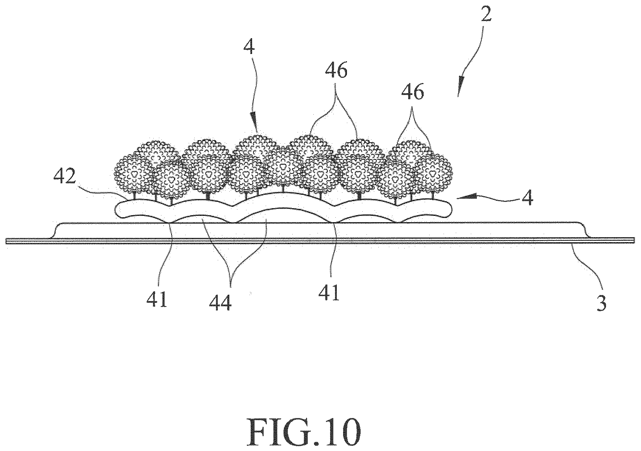

Referring to Figs. 9 and 10, a third embodiment of a sanitary napkin 2 according to this disclosure is shown to be generally identical to the second embodiment, and only differs in that: each of the absorbing units 4 further includes a plurality of cotton fluff balls 46 disposed on the absorbing portion 42, and the diameters of the cotton fluff balls 46 on one of the absorbing units 4 are different from the diameters of the cotton fluff balls 46 on an adjacent one of the absorbing units 4.

This embodiment may achieve the same effects as the second embodiment. Since the diameters of the cotton fluff balls 46 on any two adjacent absorbing units 4 are different, there are many gaps among the cotton fluff balls 46, which improve air permeability so as to prevent the generation of strange odors and thereby promote comfort of use.

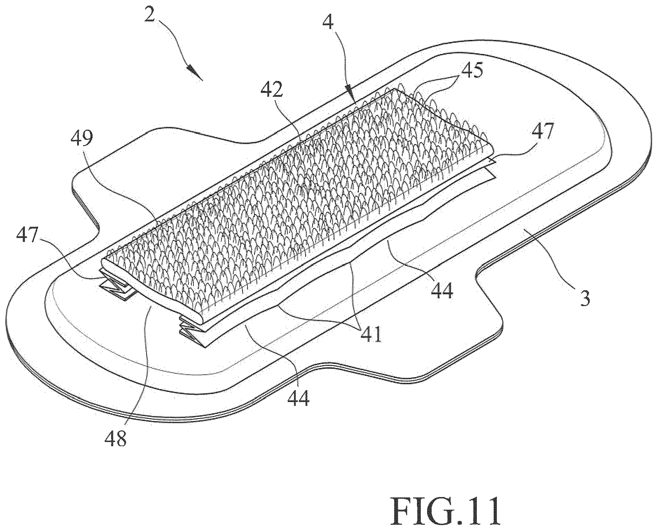

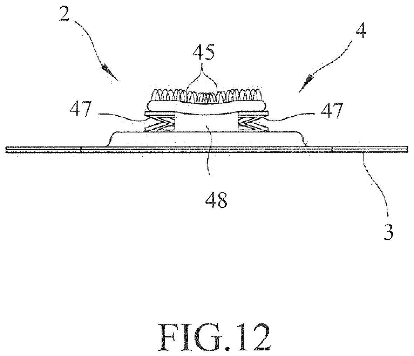

Referring to Figs. 11 and 12, a fourth embodiment of a sanitary napkin 2 according to this disclosure is shown to be generally identical to the first embodiment, and only differs in that: the connecting portions 41 are arranged in two rows extending in the longitudinal direction (X), and the absorbing portion 42 has two pleated connecting parts 47 spaced apart from each other along the width direction (Y) and extending in the longitudinal direction (X), and an absorbing part (49). Each of the pleated connecting parts (47) interconnects the absorbing part (49) and a respective one of the two rows of the connecting portions (41) . The absorbent body (32) of the substrate (3), the connecting portions (41), the pleated connecting parts (47), and the absorbing part (49) cooperatively define the aerated channels (44) each extending along the width direction (Y) and a ventilation channel (48) that extends along the longitudinal direction (X) and that is spatial communication with the aerated channels (44). This embodiment may achieve the same effects as the first embodiment, and also provide enhanced air permeability so as to promote comfort of use.

In summary, with the absorbing portion 42 which is connected to the absorbent body 32 of the substrate 3, as well as the aerated channels 44, the sanitary napkin 2 exhibits superior air permeability so as to prevent the generation of strange odors and thereby promote comfort of use.

In the description above, for the purposes of explanation, numerous specific details have been set forth in order to provide a thorough understanding of the embodiment. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects.

## Claims

1. A sanitary napkin (2) including:
a substrate (3) having a base layer (31) and an absorbent body (32) mounted protrudingly on said base layer (31), said substrate defining a longitudinal direction (X) and a width direction (Y) perpendicular to the longitudinal direction (X), and **characterized by**
at least one absorbing unit (4) including a plurality of connecting portions (41) connected to said absorbent body (32) of said substrate (3) and spaced apart among one another in the longitudinal direction (X), an absorbing portion (42) extending along the longitudinal direction (X) and connected to said absorbent body (32) of said substrate (3) through said connecting portions (41) where said absorbing portion (42) is formed as parts that are each arcuate in shape, and a plurality of cotton fluffs (45) disposed on said absorbing portion (42), said absorbent body (32) of said substrate (3), said connecting portions (41) and said absorbing portion (42) cooperatively defining a plurality of aerated channels (44) each extending along the width direction (Y).

2. The sanitary napkin (2) of Claim 1, **characterized in that** said absorbing unit (4) is formed with a plurality of perforations (43) extending through said absorbing portion (42) and being disposed in fluid communication with said aerated channels (44).

3. The sanitary napkin (2) of Claim 1 or Claim 2, **characterized by** a plurality of said absorbing units (4) that are spaced among one another along the width direction (Y).

4. The sanitary napkin (2) of Claim 3, **characterized in that** each of said absorbing units (4) further includes a plurality of cotton fluff balls (46) disposed on said absorbing portion (42), and the diameters of said cotton fluff balls (46) on one of said absorbing units (4) are different from the diameters of said cotton fluff balls (46) on an adjacent one of said absorbing units (4).

5. The sanitary napkin (2) of Claim 1 or Claim 2, **characterized in that** said absorbing unit (4) further includes a plurality of rows of cotton fluff balls (46) disposed on said absorbing portion (42), and the diameters of said cotton fluff balls (46) in one of said rows are different from the diameters of said cotton fluff balls (46) in an adjacent one of said rows.

6. The sanitary napkin (2) of Claim 1 or Claim 2, **characterized in that** said connecting portions (41) are arranged in two rows each extending in the longitudinal direction (X), and said absorbing portion (42) has two pleated connecting parts (47) spaced apart from each other along the width direction (Y) and extending in the longitudinal direction (X), and an absorbing part (49), each of said pleated connecting parts (47) interconnecting said absorbing part (49) and a respective one of the two rows of said connecting portions (41), said absorbent body (32) of said substrate (3), said connecting portions (41), said pleated connecting parts (47), and said absorbing part (49) cooperatively defining said aerated channels (44) and a ventilation channel (48) that extends along the longitudinal direction (X) and that is spatial communication with said aerated channels (44).

## Patentansprüche

1. Damenbinde (2), umfassend:
ein Substrat (3) mit einer Basisschicht (31) und einem absorbierenden Körper (32), der hervorstehend auf der Basisschicht (31) angebracht ist, wobei das Substrat eine Längsrichtung (X) und eine Breitenrichtung (Y) definiert, die senkrecht zur Längsrichtung (X) ist, und **gekennzeichnet durch**
mindestens eine Absorptionseinheit (4), die eine Vielzahl von Verbindungsabschnitten (41) umfasst, die mit dem absorbierenden Körper (32) des Substrats (3) verbunden und voneinander in der Längsrichtung (X) beabstandet sind,
einen Absorptionsabschnitt (42), der sich entlang der Längsrichtung (X) erstreckt und über die Verbindungsabschnitte (41) mit dem absorbierenden Körper (32) des Substrats (3) verbunden ist, wobei der Absorptionsabschnitt (42) als Teile gebildet ist, die jeweils eine gekrümmte Form aufweisen, und
eine Vielzahl von Baumwollflusen (45), die auf dem Absorptionsabschnitt (42) angeordnet sind, wobei der absorbierende Körper (32) des Substrats (3), die Verbindungsabschnitte (41) und der Absorptionsabschnitt (42) zusammenwirkend eine Vielzahl von belüfteten Kanälen (44) definieren, die sich jeweils entlang der Breitenrichtung (Y) erstrecken.

2. Damenbinde (2) gemäß Anspruch 1, **dadurch gekennzeichnet, dass** die Absorptionseinheit (4) mit einer Vielzahl von Perforationen (43) ausgebildet ist, die sich durch den Absorptionsabschnitt (42) erstrecken und in Fluidverbindung mit den belüfteten Kanälen (44) angeordnet sind.

3. Damenbinde (2) gemäß Anspruch 1 oder Anspruch 2, **gekennzeichnet durch** eine Vielzahl von Absorptionseinheiten (4), die voneinander entlang der Breitenrichtung (Y) beabstandet sind.

4. Damenbinde (2) gemäß Anspruch 3, **dadurch gekennzeichnet, dass** jede der Absorptionseinheiten (4) ferner eine Vielzahl von auf dem Absorptionsabschnitt (42) angeordneten Baumwollwattebällchen (46) umfasst und die Durchmesser der Baumwollwattebällchen (46) auf einer der Absorptionseinheiten (4) sich von den Durchmessern der Baumwollwattebällchen (46) auf einer benachbarten der Absorptionseinheiten (4) unterscheiden.

5. Damenbinde (2) gemäß Anspruch 1 oder 2, **dadurch gekennzeichnet, dass** die Absorptionseinheit (4) ferner eine Vielzahl von Reihen von Baumwollwattebällchen (46) umfasst, die auf dem Absorptionsabschnitt (42) angeordnet sind, und die Durchmesser der Baumwollwattebällchen (46) in einer der Reihen sich von den Durchmessern der Baumwoflwattebällchen (46) in einer benachbarten der Reihen unterscheiden.

6. Damenbinde (2) gemäß Anspruch 1 oder Anspruch 2, **dadurch gekennzeichnet, dass** die Verbindungsabschnitte (41) in zwei Reihen angeordnet sind, die sich jeweils in der Längsrichtung (X) erstrecken, und dass der Absorptionsabschnitt (42) zwei gefaltete Verbindungsteile (47), die voneinander entlang der Breitenrichtung (Y) beabstandet sind und sich in der Längsrichtung (X) erstrecken, und einen Absorptionsteil (49) aufweist, wobei jedes der gefalteten Verbindungsteile (47) den Absorptionsteil (49) und eine jeweilige der beiden Reihen der Verbindungsabschnitte (41) verbindet, wobei der Absorptionskörper (32) des Substrats (3), die Verbindungsabschnitte (41), die gefalteten Verbindungsteile (47) und die Absorptionsteile (49) zusammenwirkend die belüfteten Kanäle (44) und einen Belüftungskanal (48) definieren, der sich entlang der Längsrichtung (X) erstreckt und der in räumlicher Verbindung mit den belüfteten Kanälen (44) steht.

## Revendications

1. Serviette hygiénique (2) comprenant :
un substrat (3) comportant une couche de base (31) et un corps absorbant (32) monté de manière à être protubérant sur ladite couche de base (31), ledit substrat définissant un sens longitudinal (X) et un sens de la largeur (Y) perpendiculaire au sens longitudinal (X), et **caractérisée par**
au moins une unité absorbante (4) incluant une pluralité de sections de connexion (41) connectées audit corps absorbant (32) dudit substrat (3) et espacées les unes des autres dans le sens longitudinal (X), une section absorbante (42) s'étendant dans le sens longitudinal (X) et connectée audit corps absorbant (32) dudit substrat (3) par lesdites sections de connexion (41), ladite section absorbante (42) étant réalisée sous forme de pièces qui sont chacune de forme arquée, et une pluralité de duvets de coton (45) disposés sur ladite section absorbante (32) dudit substrat (3), lesdites sections de connexion (41) et ladite section absorbante (42) définissant coopérativement une pluralité de canaux aérés (44), chacun s'étendant dans le sens de la largeur (Y).

2. Serviette hygiénique (2) selon la revendication 1, **caractérisée en ce que** ladite unité absorbante (4) est formée d'une pluralité de perforations (43) s'étendant à travers ladite section absorbante (42) et étant disposées en communication de fluide avec lesdits canaux aérés (44).

3. Serviette hygiénique (2) selon la revendication 1 ou la revendication 2, **caractérisée par** une pluralité de dites unités absorbantes (4) qui sont espacées les unes des autres dans le sens de la largeur (Y).

4. Serviette hygiénique (2) selon la revendication 3, **caractérisée en ce que** chacune desdites unités absorbantes (4) comprend en outre une pluralité de boules de duvet de coton (46) disposées sur ladite section absorbante (42), et que les diamètres desdites boules de duvet de coton (46) sur une desdites unités absorbantes (4) sont différents des diamètres desdites boules de duvet de coton (46) sur une unité adjacente parmi lesdites unités absorbantes (4).

5. Serviette hygiénique (2) selon la revendication 1 ou la revendication 2, **caractérisée en ce que** ladite unité absorbante (4) comprend en outre une pluralité de rangées de boules de duvet de coton (46) disposées sur ladite section absorbante (42), et que les diamètres desdites boules de duvet de coton (46) d'une desdites rangées sont différents des diamètres desdites boules de duvet de coton (46) d'une rangée adjacente parmi lesdites rangées.

6. Serviette hygiénique (2) selon la revendication 1 ou la revendication 2, **caractérisée en ce que** lesdites sections de connexion (41) sont disposées en deux rangées, chacune s'étendant dans le sens longitudinal (X), et que ladite unité absorbante (42) comporte deux pièces de connexion plissées (47) espacées l'une de l'autre dans le sens de la largeur (Y) et s'étendant dans le sens longitudinal (X), et une pièce absorbante (49), chacune desdites pièces de connexion plissées (47) interconnectant ladite pièce absorbante (49) et une rangée respective parmi les deux rangées desdites sections de connexion (41), ledit corps absorbant (32) dudit substrat (3), lesdites sections de connexion (41), lesdites pièces de connexion plissées (47), et ladite pièce absorbante (49) définissant coopérativement lesdits canaux aérés (44) et un canal de ventilation (48) qui s'étend dans le sens longitudinal (X) et qui est en communication spatiale avec lesdits canaux aérés (44).
